# EUROPEAN PATENT SPECIFICATION

(11) **EP 1 641 499 B1**
(45) Date of publication and mention of the grant of the patent: **19.12.2012**
(21) Application number: 04742989.9
(22) Date of filing: 21.06.2004
(51) Int. Cl.: A61L 15/28, A61L 15/22, A61L 15/32, A61L 15/42, A61L 15/56

(54) **ANTIOXIDANT WOUND DRESSING MATERIALS**
ANTIOXIDATIVES WUNDVERBANDMATERIAL
MATERIAUX DE PANSEMENT ANTIOXYDANTS

(30) Priority: 20.06.2003 GB 0314454; 04.08.2003 US 491991 P; 18.11.2003 GB 0326844
(43) Date of publication of application: 05.04.2006
(73) Proprietor: Systagenix Wound Management IP Co. BV., 1077 XX Amsterdam (NL)
(72) Inventor: CULLEN, Breda, Mary, North Yorkshire BD23 1HR (GB); ADDISON, Deborah, Via Lancaster LA2 8HB (GB); GREENHALGH, David, North Yorkshire BD23 2LG (GB)
(74) Representative: James, Anthony Christopher W.P.
(86) International application number: PCT/GB2004/002636
(87) International publication number: WO 2004/112850

(56) References cited:
- EP-A- 0 560 014
- EP-A- 1 245 239
- WO-A-01/24839
- WO-A-02/30478
- WO-A-94/13333
- DE-A- 19 712 699

## Description

The present invention relates to antioxidant wound dressing material, processes suitable for the preparation of such materials, and to the use or such wound dressing materials.

Concentrations of reactive oxygen species such as hydroxyl radicals (-OII), singlet oxygen (¹O₂), hydroperoxyl radical (OOII), superoxideradical anions (-O₂⁻), and hydrogen peroxide (H₂O₂) can rise in damaged tissues, producing a condition known as oxidative stress. The presence of a low level of reactive oxygen species may be advantageous in the early stages of wound healing by both attracting and activating macrophages which engulf and kill bacteria and release cytokines and growth factors. However, prolonged and more severe oxidative stress may delay healing because it will produce chronic inflammation, divert available energy supply towards antioxidant defence at the expense of tissue reconstruction, and increase levels of matrix metalloproteinases which cause tissue breakdown, In more severe cases, elevated levels of reactive oxygen species can give rise to hydrogen peroxide-induced senescence or apoptosis (that is, programmed cell death) or tissue necrosis (that is, uncontrolled cell death and therefore permanent tissue damage).

Under mild oxidative stress, it is thought that hydrogen peroxide (H₂O₂) is the dominant species present, being formed rapidly from superoxide by the enzyme superoxide dismutase This enzyme-mediated disputation reaction also minimises the production of singlet oxygen that can arise when superoxide is produced too rapidly and therefore has the opportunity to dismutate spontaneously without enzyme assistance. Rapid enzyme-mediated dismutation of superoxide also minimises levels of hydroperoxyl radical, the unionised form of superoxide. Levels of hydrogen peroxide are in turn kept low by the actions of catalase and glutathione peroxidase. Thus, under mild oxidative stress conditions when hydrogen peroxide levels are slightly raised (around 10⁸ to 10⁴ Molar), it has been found that the rate of cell proliferation in fibroblast cultured is stimulated.

Accordingly, the healing of chronic wounds may be assisted by the use of antioxidant wound dressings that react specifically with excess reactive oxygen species such as those listed above and hence reduce the level of oxidative stress.

US-A-5667501 describes compositions comprising chemically modified polymers grafted with chemical groups that confer antioxidant activity as measured by a diphenylpicrylhydrazyl (DPPH) test and that also generate low levels of hydrogen peroxide by reaction with molecular oxygen in the wound bed to simulate macrophage activity and fibroblast proliferation. The compositions may be used to promote the healing of chronic wounds. Preferably, the polymer is a polymer bearing hydroxyl, carbonyl or amide functional groups, or a polysaccharide bearing hydroxyl functional groups, said functional groups having been converted to derivatives that are persistent free radicals of precursors of persistent free radicals, that is to say they are free radical scavenging antioxidant groups.

US-A-5612321 describes compositions comprising polysaccharides grafted with antioxidants on at loast one hydroxyl group of the polysaccharide. The compositions may be used inter alia to promote the healing of chronic wounds. Preferably, the polysaccharide is hyaluronic acid and the antioxidant group comprises a phenol group.

The above antioxidant wound dressing materials are made by multi-step chemical reactions to achieve covalent bonding of antioxidant moieties, such as hydroquinones or benzimidazole derivatives, to the polymeric substrate materials. A need remains for a more simple and inexpensive route to antioxidant wound dressing materials.

Wound dressings that contain antimicrobial agents for the treatment of wound infection are also known. Currently preferred antimicrobial agents for use in wound dressings are certain antiseptics such as chlorhexidine and triclosan, and silver, whether in the form of thin films, nanoparticles, or colloidal silver. Chemical compounds of silver are also useful as antimicrobials. For example, the following complex silver salts are favored for use against sensitive and resistant bacterial strains: silver sulfadiazine, silver norfloxacinate, silver pipemidate, kaolin silver, silver EDTAate, silver thiosalicylate and silver imidazolium chloride.

WO91/11206 describes the use of silver alginate salts in wound dressings. WO87/05517 describes silver salts of hyaluronic acid that may be used as or in antimicrobial wound dressings. These materials tend not to be stable in the presence of light. The silver undergoes photochemical reduction to metallic silver, causing a darkening of the materials over time.

WO02/43743 describes light-stabilized antimicrobial wound dressing materials in which silver salts are stabilized by the addition of a photostabitizer selected from the group consisting of ammonium salts, thiosulfates, metal chlorides and peroxides. WOP7/02038 describes light-stabilized antimicrobial wound dressing materials in which silver salts are stabilized by the addition of a polyether polymer. Such photostabilizers are of limited effectiveness, and will tend to be extracted from the dressing material by wound fluid.

A need therefore remains for improved antimicrobial dressings containing light-stabilized silver compounds.

GB-A-619165 describes calcium alginate fibers for use as wound dressings. A bacteriostatic or antiseptic compound, such as an acridine derivative or eusol, may be applied to the filaments after they are formed, or may be added to the solution from which the filaments are drawn. Calcium alginate is not bioabsorbable.

EP-A-0368253 describes chitosan films, rods, sheets, medicated bandages, patches and the like that have been fabricated from solutions or mixtures of certain chitosan derivatives in combination with antimicrobial agents. There is no disclosure of dyeing a bioabsorbable wound dressing material with an antioxidant dye.

In a first aspect, the present invention provides a wound dressing material as set out in claim 1*.*

The term "dyed" refers to a solid material that has been surface-treated while in the solid state with a dye to bind the dye to the surface thereof. That is to say, a solid material that has been subjected to post treatment with a dye after solidification. It has been found that bioabsorbable substrate materials such as oxidized regenerated cellulose have excellent avidity for antioxidant dyes such as aniline and acridine dyes. This enables controlled amounts of the dyes to be fixed onto the substrate materials in a simple and inexpensive dyeing step. It has further been found that the resulting dyed materials retain the antioxidant properties of the dyestuff, thereby making them excellent candidates for the treatment of chronic wounds and other wounds characterised by elevated levels of oxygen free radicals. The materials also have useful antimicrobial properties, in particular against gram-positive and sometimes also gram-negative bacteria. The gradual breakdown of the bioabsorbable material in the wound achieves sustained release of effective amounts of antimicrobial.

The term "bioabsorbable substrate material" refers to a solid material that is fully degraded and absorbed *in vivo* in the mammalian body. The term therefore does not encompass cellulose or conventional textile materials. The substrate material is usually not water soluble, but it may be water swellable.

The substrate comprises (and may consist essentially of) a solid bioabsorbable material selected from the group consisting of collagens, chitosans, oxidized regenerated celluloses, and mixtures thereof.

Oxidized cellulose is produced by the oxidation of cellulose, for example with dinitrogen tetroxide. This process converts primary alcohol groups on the saccharide residues to carboxylic acid group, forming uronic acid residues Within the cellulose chain. The oxidation does not proceed with complete selectivity, and as a result hydroxyl groups on carbons 2 and 3 are occasionally converted to the keto form. These ketone units introduce an alkali labile link, which at pH7 or higher initiates the decomposition of the polymer via formation of a lactone and sugar ring cleavage. As a result, oxidized cellulose is biodegradable and bioabsorbable under physiological conditions.

The preferred oxidized cellulose for practical applications is oxidized regenerated cellulose (ORC) prepared by oxidation of a regenerated cellulose, such as rayon. It has been known for some time that ORC has haemostatic properties, and that application of ORC fabric can be used to reduce the extent of post-surgical adhesions in abdominal surgery.

The oxidized regenerated cellulose (ORC) can be obtained by the process described in US-A-3122479. This material offers numerous advantages including the features that it is biocompatible, biodegradable, non-immunogenic and readily commercially available. ORC is available with varying degrees of oxidation and hence rates of degradation. The ORC may be used in the form of insoluble fibers, including woven, non-woven and knitted fabrics.

In certain embodiments, the oxidized cellulose is in the form of particles, such as fiber particles or powder particles, preferably dispersed in a suitable solid or semisolid topical medicament vehicle. In particular, the materials preferably contain ORC fibers, wherein a volume fraction of at least 80% of the fibers have lengths in the range of 20µm to 1000µm. Such a size distribution can be achieved, for example, by milling an ORC cloth, followed by sieving the milled powder to remove fibers outside the range. Preferably, the average (mean by volume) length of the ORC fibers is in the range 25µom to 450µm. The election of ORC fiber lengths in this range results in easy mixing of the ORC and other component, and highly homogeneous products. The ORC is more thoroughly complexed with the other components, which results in enhanced therapeutic properties of the sponge.

Preferably, the oxidised cellulose has an average molecular weight greater than 50,000. Such oxidised cellulose is substantially insoluble in wound fluids, but will undergo very gradual breakdown into bioresorbable fragments at physiological pH. The oxidized cellulose may be in its free acid form, or it may be neutralized. For example, the present invention encompasses the use of partially or completely neutralized materials as described in EP-A-0437095. For example, the ORC may be partially neutralised by a silver salt of a weak acid, such as silver acetate, as described in more detail below.

In certain embodiment of the present invention, the oxidized cellulose is complexed with collagen and/or chitosan to form structures of the kind described in WO98/00180, WO98/00446, EP-A-1153622 or WO2004/026200. For example, the oxidized cellulose may be in the form of milled ORC fibres that are dispersed in a freeze dried collagen sponge. This provides for certain therapeutic and synergistic effects arising from the complexation with collagen.

Where used, the collagen in the materials of the present invention may be any collagen, including Type I, Type II or Type III collagen, natural fibrous collagen, atelocollagen, partially hydrolysed collagens such as gelatin, and combinations thereof. Natural fibrous collagen, for example of bovine origin, is suitable. For example, the collagen prepared from bovine hide is a combination of Type I collagen (85%) and Type III collagen (15%).

Where used, the chitosan in the materials of the present invention is usually derived from chitin. Chitin is a natural biopolymer composed of N-acetyl-D-glucosamine units. Chitin may be extracted from the outer shell of shrimps and crabs in known fashion. The chitin is then partially deacetylated, for example by treatment with 5M-15M NaOH, to produce chitosan. Complete deacetylation of the chitin is not a practical possibility, but preferably the chitosan is at least 50% deacetylated, more preferably at least 75% deacetylated. Chitosan has been employed for wound treatment in various physical forms, e.g. as a solution/gel; film/membrane; sponge; powder or fiber. Chitosan in the free base form is swellable but not substantially soluble in water at near-neutral pH, but soluble in acids due to the presence of ammonium groups on the chitosan chain. The solubility of the chitosan may be reduced by cross linking, for example with epichlorhydrin. Typically, the average molecular weight of the chitosan as determined by gel permeation chromatography is from 105 to 106.

In particular embodiments, the substrate comprises (and may consist essentially of) a mixture of: (a) collagen and/or chitosan; and (b) oxidized regenerated cellulose, for example in a dry weight ratio range of from 90:10 to 10:90 of collagen/chitosan ORC, preferably from 75:25 to 25:75, and particularly from 60:40 to 40:60.

The wound dressing material according to the present invention may also contain a silver salt. Some of the silver may be present as metallic silver, but preferably a major part of the silver is present as a silver salt, preferably as a substantially colorless silver salt. Preferably, the silver in the material consists essentially of silver salts, more preferably as substantially colorless silver salts. Preferably, the amount of silver (as silver ions and metallic silver) in the materials according to the present invention is from 0.01wt% to 5wt.%, more preferably from 0.1wt% to 2wt.%, and most preferably 0.1wt.% to 1wt.%, most preferably about 0.3wt.%. Lesser amounts of silver could give insufficient antimicrobial effect. Greater amounts of silver could give riso to antiproliferative effects on wound healing cells.

The silver may be introduced by treating a bioabsorbable substrate material with a silver salt or compound dissolved or dispersed in water or an organic solvent such as ethanol, for example as described in WO02/43743. Suitable compounds include silver oxide, silver chromate, silver allantoinate, silver borate, silver glycerolate, silver nitrate, silver acetate, silver chloride, silver sulfate, silver lactate, silver bromide, silver iodide, silver carbonate, silver citrate, silver laurate, silver deoxycholate, silver salicylate, silver p-arminobenzoate, silver p-aminosalicylate, and mixtures thereof. Preferably, the silver is not present as silver sulfadiazine.

In preferred embodiments, the silver may be complexed to the substrate material The term "complex" refers to an intimate mixture at the molecular scala, preferably with ionic or covalent bonding between the silver and the polymer. The complex preferably comprises a salt formed between an anionic polymer and Ag⁺. Suitably, the anionic polymer is a polycarboxylate or a polysulfated polysaccharide. Suitably, the anionic polymer comprises an anionic polysaccharide. Suitable anionic polysaccharides include hyaluronates, pectins, carrageenans, xanthan gums, sulfated polysaccharides such as dermatan sulfate or sulfated dextrans, and oxidized celluloses.

The complex of an anionic polymer and silver can be made by a method comprising the step of treating an anionic polymer with a solution of a silver salt. Preferably, the solution is an aqueous solution. Preferably, the anionic polymer is substantially insoluble in water at pH7, and the treatment is therefore carried out on the polymer in the solid state. For example, the polymer may be in the form of solid fibers, sheet, sponge or fabric. In certain embodiments, the anionic polymer is a salt and the treatment therefore can be regarded as an ion exchange. In other embodiments, the anionic polymer is at least partly in free acid form, in which case the silver salt is preferably a salt of a weak acid, for example silver acetate, whereby the anionic polymer is at least partially neutralised by the silver salt. Similar processes are described in EP-A-0437095.

The neutralization reaction can be carried out in water or alcohol alone but is preferably carried out in mixtures of water and alcohols. The use of a mixture of water and alcohol provides good solubility for the weak acid salts via the water, and the alcohol prevents the anionic polymer from excessively swelling, distorting and weakening during the neutralization. Thus the physical properties of the material are retained. Methanol is the preferred alcohol because many of the above-mentioned salts have good solubility in this alcohol in combination with water. Preferably, the alcohol to water ratio has a range of 4:1 to 1:4 If the solution becomes too rich in alcohol, some salts may no longer be soluble particularly if the alcohol is other than methanol. If the solution becomes too rich in water, some swelling of the polymer will occur as neutralization takes place and there will be some loss in physical properties such as in the tensile strength of the polymer. Other useful alcohols include, for example, ethyl alcohol, propyl alcohol and isopropyl alcohol.

The use of a mild neutralizing agent such as silver acetate allows for control of the degree of neutralization. Use of stoichiometric and chemically equivalent amounts of neutralizing agent and carboxylic acid on the anionic polymer does not produce a 100% neutralized polymer as would be produced with strong irreversible reactions with bases such as sodium hydroxide, sodium carbonate, sodium bicarbonate and ammonium hydroxide.

Anionic polymers behave as an ion exchange and will pull out of solution the silver cation of any silver salt that is passed over them. The by-product of this exchange is an acid from the salt and by using a salt of a weak organic acid, a weak acid such as acetic acid is produced which does no damage to the polymer Using salts of strong acids such as sodium chloride or sodium sulfate produces hydrochloric acid or sulfuric acid by-products respectively, and these strong acids can cause damage such as depolymerization of the polymer

When using silver salts of weak acids, the silver ion is exchanged for a proton on the polymer and part of the salt is converted to weak acid. The mixture of acid and salt in the solution results in a buffered solution which maintains a fairly constant pH and controls the degree of neutralization. An equilibrium reaction is established whereby the silver ions are bound to the acid portion of the polymer and also to the salt molecules. This partitioning of the silver ions prevents the neutralization of the polymer from going to cornpletion.

Using a stoichiometric amount of, for example, silver acetate brings about a 65-75% degree of neutralization of the carboxylic acid groups on an oxidized cellulose * polymer. This control of pH by creating a self generating buffered solution and the use of methanol to control the swelling of the material, leads to a partially neutralized material in which the physical properties, e.g. tensile strength and shape of the polysaccharide, are preserved.

The amount of silver salt used is generally about equal to or up to twice the stoichiometric amount of acid content of the polymer. Alternatively, a second charge of a stoichiometric amount of silver salt can be used if the reaction is recharge with fresh solvent and salt after the first charge reaches a constant pH. The material with elevated pH is then washed to remove the excess silver salt and ions therefrom.

In some embodiments, at least a portion of the wound dressing material comprises a collagen complexed with silver. This can be achieved by treating a Collagen with a solution of a silver salt. The silver salt may for example be silver acetate or silver nitrate at a concentration of 0.01 molar to 1 molar. The treatment is preferably carried out at a pH of from 5 to 9. It is thought that the silver complexes primarily to the nitrogen-containing side chains of the collagen amino acids, in particular to lysine, hydroxylysine, asparagine, glutamine and arginine. The silver could also bind to the sulfhydryl groups of methionine and cysteine residues, where present, and to carboxyl groups of aspartate and glutamate.

Preferably the amount of silver in the collagen complex is from 0.01 to 30% by weight based on the weight of the collagen, more preferably from 0.1% to 20%, more preferably from 2% to 10% by weight. Preferably, the amount of silver-collagen complex in the wound dressing material is from 0.1 to 10 wt.%, more preferably from 0.1 to 2wt. %. In any case, the total amount of silver in the wound dressing material is generally as specified above.

It will be appreciated that the complexes of silver with substrate materials described above may be prepared with a relatively high silver content, for example greater than 5wt.%, and then diluted with further substrate material (the same or different) to achieve the desired overall silver content of from 0.01wt.% to 5wt.%, preferably from 0.2wt.% to 2wt.%.

The solid bioabsorbable substrate comprises (or consists essentially of) a freeze-dried sponge or a solvent-dried sponge, for example as described hereinbefore.

The solid bioabsorbable substrate is typically in sheet form, for example a sheet of material having an area of from 1cm² to 400cm², in particular from 2cm² to 100cm². The basis weight of the sheet is typically from 100g/m² to 5000g/m², for example from 400g/m² to 2000g/m².

The solid bioabsorbable substrate material may make up at least 50% by weight of the wound dressing material, for example at least 75% by weight or at least 90% by weight.

The term "dyestuff" refers to a material that is useful as a colorant for textile materials, that is to say an organic compound that is strongly light-absorbing in the visible region 400-700nm. In certain embodiments, the antioxidant dyestuff is selected from the group consisting of aniline dyes, acridine dyes, thionine dyes, bis-naphthalene dyes, thiazine dyes, azo dyes, anthraquinone dyes, and mixtures thereof. For example, the antioxidant dyestuff may be selected from the group consisting of gentian violet, aniline blue, methylene blue, crystal violet, acriflavine, 9-aminoacridine, acridine yellow, acridine orange, proflavin, quinacrine, brilliant green, trypan blue, trypan red, malachite green, azacrine, methyl violet, methyl orange, methyl yellow, ethyl violet, acid orange, acid yellow, acid blue, acid red, thioflavin, alphazurine, indigo blue, methylene green, and mixtures thereof.

The dyestuffs also stabilize silver salts present in the material against photochemical decomposition, in particular against photochemical reduction to metallic silver, by absorbing light near the surface of the material. The dyestuffs further trap photochemically generated free radicals that could otherwise react with the silver. In this way the dyestuffs can act as photochemical descnsitisers. In addition to the conventional dyestuffs listed above, medically acceptable organic desensitisers of the kind used in photography may be Suitable for use as the dyestuffs in the materials of the present invention.

The dyestuff may be present in the wound dressing material according to the invention in an amount of from 0.05% to 5wt.%, typically 0.2 to 2wt % based on the dry weight of the material.

The wound dressing material may also comprise up to 20% by weight, preferably less than 10% by weight of water. The material may also contain 0-409% by weight, preferably 0-25% by weight of a plasticiser, preferably a polyhydric alcohol such as glycerol, The material may also comprise 0-10% by weight, preferably 0-5% by weight of one or more therapeutic wound healing agents, such as non-steroidal anti-inflammatory drugs (e.g. acetaminophen), steroids, antibiotics (e.g. penicillins or streptomycins), antiseptics (e.g. silver sulfadiazine or chlorhexidine), or growth factors (e.g. fibroblast growth factor or platelet derived growth factor). All of the above percentage are on a dry weight basis.

The wound dressing material according to the present invention is preferably sterile and packaged in a microorganism-impermeable container.

Preferably, the material according to the present invention has a free radical activity, that is to say an antioxidant activity, of at least 15% in the diphenylpierylhydrazyl (DPPH) test, measured as percentage reduction in absorbance at 524nm after 4 hours of a 0,5%w/v dispersion of the polysaccharide in 10⁻⁴M DPPH, as described further hereinbelow in Procedure 1. Preferably the percentage reduction in absorbance in the DPPH test (after correction for any absorbance by the dye) is at least about 25%, more preferably at least about 50%, and most preferably at least about 75%.

Allernatively or additionally, the material according to the present invention may exhibit antioxidant activity as measured by its ability to inhibit the oxidation of ABTS (2,2' azino-di-[3-ethylbenzthiazoline sulphonate]) by a peroxidase.

Preferably, the material according to the present invention will absorb water or wound fluid and hence become wet, swell or become a gelatinous mass but will not spontaneously dissolve or disperse therein. That is to say, it is hydrophilic but has a solubility of preferably less than about 1g/liter in water at 25°C. Low volubility renders such materials especially suitable for use as wound dressings to remove reactive oxygen species from the wound fluid.

The antioxidant and antimicrobial properties of the material according to the present invention suggest applications in a range of medical applications, including the treatment of acute surgical and traumatic wounds, burns, fistulas, venous ulcers, arterial ulcers, pressure sores (otherwise known as decubitus ulcers), diabetic ulcers, ulcers of mixed aetiology, and other chronic or necrotic wounds and inflammatory lesions and disorders. The materials according to the present invention are intended for the treatment of both infected and non-infected wounds (that is to say wounds showing no clinical signs of infection).

Accordingly, in a second aspect, the present invention provides the use of a material according to the present invention for the preparation of a medicament for the treatment of a wound. Preferably, the wound is a chronic wound. More preferably, the chronic wound is selected from the group consisting of ulcers of venous, arterial or mixed aetiology, decubitus ulcers, or diabetic ulcers. Preferably, the material is used as an antioxidant to reduce oxidative stress in the wound environment and thereby to promote wound healing.

Treatment of a wound in a mammal can comprise applying thereto a therapeutically effective amount of a material according to the present invention. Preferably, the wound is a chronic wound.

In a third aspect, the present invention provides a wound dressing comprising an antioxidant wound dressing material according to the present invention.

The wound dressing is preferably in sheet form and comprises an active layer of the material according to the invention. The active layer would normally be the wound contacting layer in use, but in some embodiments it could be separated from the wound by a liquid-permeable top sheet. Preferably, the area of the active layer is from 1cm² to 100 cm², more preferably from 4cm² to 100cm².

Preferably, the wound dressing further comprises a backing sheet extending over the active layer opposite to the wound facing side of the active layer. Preferably, the backing sheet is larger than the active layer such that a marginal region of width 1mm to 50mm, preferably 5mm to 20mm extends around the active layer to form a so-called island dressing. In such cases, the backing sheet is preferably coated with a pressure sensitive medical grade adhesive in at least its marginal region.

Preferably, the backing sheet is substantially liquid-impermeable. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapor transmission rate (MVTR) of the backing sheet alone of 300 to 5000 g/m²/24hrs, preferably 500 to 2000 g/rn²/24hrs at 37.5 °C at 100% to 10% relative humidity difference. The backing sheet thickness is preferably in the range of 10 to 1000 micrometers, more preferable 100 to 500 micrometers. It has been found that such moisture vapor transmission rates allow the wound under the dressing to heal under moist conditions without causing the skin surrounding the wound to macerate.

Suitable polymers for forming the backing sheet include polyurethanes and poly alkoxyalkyl acrylates and methacrylates such as those disclosed in GB-A-1280631. Preferably, the backing sheet comprises a continuous layer of a high density blocked polyurethane foam that is predominantly closed-cell. A suitable backing sheet material is the polyurethane film available under the Registered Trade Mark ESTANE 5714F.

The adhesive (where present) layer should be moisture vapor transmitting and/or patterned to allow massage of water vapor therethrough. The adhesive layer is preferably a continuous moisture vapor transmitting, pressure-sensilive adhesive layer of the type conventionally used for island-type wound dressings, for example, a pressure sensitive adhesive based on acrylate ester copolymers, polyvinyl ethyl ether and polyurethane as described for example in GB-A-1280631. The basis weight of the adhesive layer is preferably 20 to 250 g/m², and more preferably 50 to 150 g/m². Polyurethane-based pressure sensitive adhesives are preferred.

Further layers of a multilayer absorbent article may be built up between the active layer and the protective sheet. For example, these slayers may comprise an absorbent layer between the active layer and the protective sheet, especially if the dressing is for use on exuding wounds. The optional absorbent layer may be any of the layers conventianolly used for absorbing wound fluids, serum or blood in the wound healing art, including gauzes, nonwoven fabrics, superabsorbcnts, hydrogels and mixtures thereof. Preferably, the absorbent layer comprises a layer of absorbent foam, such as an open celled hydrophilic polyurethane foam prepared in accordance with EP-A-0541391. In other embodiments, the absorbent layer may be a nonwoven fibrous web, for example a carded web of viscose staple fibers. The basis weight of the absorbent layer may be in the range of 50-500g/m², such as 100-400g/m². The uncompressed thickness of the absorbent layer may be in the range of from 0.5mm to 10mm, such as 1mm to 4mm. The free (uncompressed) liquid absorbency measured for physiological saline may be in the range of 5 to 30 g/g at 25°. Preferably, the absorbent layer or layers are substantially coextensive with the active layer.

The wound facing surface of the dressing is preferably protected by a removable cover sheet. The cover sheet is normally formed from flexible thermoplastic material. Suitable materials include polyesters and polyolefins. Preferably, the adhesive- facing surface of the cover sheet is a release surface. That is to say, a surface that is only weakly adherent to the active layer and the adhesive on the backing sheet to assist peeling of the adhesive layer from the cover sheet. For example, the cover sheet may be formed from a non-adherent plastic such as a fluoropolymer, or it may be provided with a release coating such as a silicone or fluoropolymer release coating.

Typically, the wound dressing according to the present invention is sterile and packaged in a microorganism-impermacable container.

In a further aspect, the present invention provides a method of manufacture of an antioxidant wound dressing material, comprising the step of dyeing a bioabsorbable substrate material with a suitable dye. The method preferably further comprises treating the substrate material with a silver salt dissolved or dispersed in water or an organic solvent.

The method according to the present invention may be used to prepared a wound dressing according to the present invention.

The method of the present invention may comprise dyeing a substrate material in sheet form, for example a sponge sheet of the substrate material by immersing it in a dye bath, followed by washing to remove unbound dye and drying. In other embodiments, the substrate material may be dyed while it is in fibrous or particulate form, followed by forming the material into a sheet. For example, a slurry of fibers or particles of the substrate material may be treated with dye, and then freeze-dried to form a dyed sponge. The silver treatment may be carried out after the step of removing unbound dye.

It will be appreciated that any feature or embodiment that is described herein in relation to any one aspect of the invention may also be applied to any other aspect of the invention equally.

Certain specific embodiments of the present invention will now be described further in the following examples.

### Example 1

An antioxidant wound dressing material based on a collagen/ORC freeze-dried sponge material is prepared as follows.

The collagen component is prepared from bovine corium as follows. Bovine corium is split from cow hide, scraped and soaked in sodium hypochlorite solution (0.03% w/v) to inhibit microbial activity pending further processing The curium is then washed with water and treated with a solution containing sodium hydroxide (0.2% w/v) and hydrogen peroxide (0.02% w/v) to swell and sterilize the corium at ambient temperature. The corium splits then undergo an alkali treatment step in a solution containing sodium hydroxide, calcium hydroxide and sodium bicarbonate (0.4% w/v, 0.6% w/v and0.05% w.v, respectively) at pH greater than 12.2, ambient temperature, and for a time of 10-14 days, with tumbling, until an amide nitrogen level less than 0.24mmol/g is reached. The corium splits then undergo an acid treatment step with 1% hydrochloric acid at ambient temperature and pH 0.8-1.2. The treatment is continued with tumbling until the corium splits have absorbed sufficient acid to reach a pH less than 2.5. The splits are then washed with water until the pH value of corium splints reaches 3.0-3.4. The corium splits are then comminuted with ice in a bowl chopper first with a coarse comminution and then with a fine comminution setting. The resulting paste, which is made up in a ratio of 650g of the corium splits to 100g of water, as ice, is frozen and stored before use in the next stage of the process. However, the collagen is not freeze-dried before admixture with the ORC & other components in the next stage.

The ORC component of the freeze dried pad is prepared as follows. A SURGICEL cloth (Johnson & Johnson Medical, Arlington) is milled using a rotary knife cutter through a screen-plate, maintaining the temperature below 60°C.

Methylene blue, an acidic dye, was incorporated by dissolving an appropriate amount of the dye in 0.05M acetic acid and adding to the collagen paste with the milled ORC powder to obtain a final solids concentration of 1%. Samples were made in which the dye was incorporated at the following concentrations in the slurry; 0% (reference example), 1mg/ml, 0.5mg/ml and 0.1mg/ml.

The resulting slurries were poured to a depth of 3mm in petri dishes, placed onto freezer shelves where the temperature has been preset to -40°C. The freeze-drier programme was then initiated to dry and dehydrothermally cross-link the collagen and ORC to form sponge pads. On completion of the cycle, the vacuum was released, sponge samples were then packaged, and sterilized by cobalt 60 gamma irradiation.

### Example 2

The procedure of Example 1 was followed, but replacing the methylene blue dye by crystal violet, a basic dye. The crystal violet was incorporated at the following concentrations in the slurry: 0% (reference example), 1mg/ml, 0.5mg/ml and 0.1mg/ml.

### Example 3

The procedure of Example 1 was followed, but replacing the methylene blue dye by flavin 3,6 Diaminoacridine hemisulfate, a basic dye. The flavin was incorporated at the following concentrations in the slurry: 0% (reference example), 1mg/ml, 0.5mg/ml and 0.1mg/ml.

### Example 4

The procedure of Example 1 was followed, but replacing the methylene blue dye by a mixture of methylene blue and flavin 3,6-Diaminoacridine hemisulfate, each dye being incorporated in the slurry at a concentration of 0.5mg/ml.

### Example 5

The procedure of Example 1 was followed, but replacing the methylene blue dye by a mixture of crystal violet and flavin 3,6-Diaminoacridine hemisulfate, each dye being incorporated in the slurry at a concentration of 0.5mg/ml.

### Example 6

The procedure of Example 1 was followed, but replacing the methylene blue dye by a mixture of crystal violet and methylene blue, each dye being incorporated in the slurry at a concentration of 0.5mg/ml.

### Examples 7-12

Silver was incorporated into the wound dressing materials made as described in Examples 1-6 by dissolving silver acetate in 0.05M acetic acid and adding the solution to the ORC/collagen slurry in an amount sufficient to produce a final slurry containing 1 wt.% silver on a total solids basis.

The sponges according to the invention obtained in Examples 1 to 12 all showed stable absorption of the dyes. The sponges could be soaked in serum at 25°C for a number of days and remained coloured at all times. Depending on concentration of dye added there was an initial release of the excess dye and then a gradual release as the sponges began to degrade.

### Procedure 1

The ability of the wound dressing materials to react with and remove oxygen containing free radicals is assessed by the DPPH test described in WO94/13333. The test is adapted from that described by Blois M.S. in Nature 181; 1199 (1958), and Banda P.W. et al., in Analytical Letters 7: 41 (1974).

Briefly, the wound dressing material under test (2.5mg: 5mg; & 25mg sample sizes) was suspended in 2.5ml of 0.1M pH 1.0 phosphate buffer. A solution of diphenylpicrylhydrazyl (DPPII) in methanol (10⁻¹ M) was added in an amount of 2.5 ml and the mixture was shaken and stored in the dark at 20°C. The simples were assessed by measurement of their light absorbance at 524nm over 6 hours in comparison with a control, particular attention being paid to the figure after 4 hours. The percentage reduction of absorbance relative to the control after 4 hours gives the DPPH test value, with a reproducibility generally of ±5%. This value may conveniently be expressed in terms of a simple reduction in absorbance units (AU) relative to the control.

Ascorbic acid, a well-known antioxidant, provides a useful positive control substance for comparative purposes. Freeze dried sponges of chitin/chitosan and hydroxyethyl cellulose were used as negative controls.

Application of this test to the materials according to the present invention of Examples 1-6 resulted in DPPH test values of 80 - 90% for the positive control (10⁻⁴M). In contrast, the negative controls chitin/chitosan and hydroxyethyl cellulose exhibited much lower DPPH values of less than 15%. The collagen/ORC without any added dye exhibited some activity in the DPPH test, indicating that ORC itself has some antioxidant properties. The dyed materials according to the present invention exhibited significantly higher activity in the DPPH test than collagen/ORC alone, consistent with antioxidant activity of the dyes.

### Procedure 2

The bactericidal activity of the sponges prepared in Examples 7 to 12 is tested on *pseudomonas Aeruginosa* and *staphylococcus Aureus* by looking at zone of inhibition.

Six 2cm x 2cm squares of each sample are cut out in sterile conditions. On day one of the experiment, cultures of both *Pseudomonas aeruginosa* (ATCC 27853 and various PSI strains) and *Staphylococcus aureus* (provided by the Dept of Clinical Microbiology and Pathology) are incubated aerobically at 37°C for 24 hours on Diagnostic Sensitivity Agar (DSA). After 24 hours test samples are each placed on a DSA plate and immediately wetted with 0.5mls of a buffer solution. Three squares of sample are placed on plates inoculated with *Pseudomonas aeruginosa* and three are placed on plates inoculated with *Staphylococcus aureus.* The plates are then incubated at 37°C for 24 hours. The zone of inhibited growth around the sample is then measured using calipers, and the test sample is placed on a new inoculated DSA plate. A swab test is carried out on the area beneath the sample to determine if the sample is bacteriostatic if not bactericidal by smearing the swab on a DSA plate and incubating it for 24 hours and then examining the growth. The samples are transferred onto fresh inoculated plates with the above procedure being carried out every 24 hours for 72 hours as long as the samples remain intact.

As a negative control, a freeze dried sponge of 45%ORC/55%collagen without any silver or dye was tested. A commercially available silver-containing antimicrobial dressing (ACTICOAT, registered trade mark of Smith & Nephew) and silver nitrate solution (0.5%) were used as positive controls and zones of inhibition were observed for both over the test period.

It is found that significant bactericidal effects are observed against *Staphylococcus aureus* and *Pseudomonas Aeruginosa* for tho materials according to the invention. The performance of the materials containing 1% silver and above is expected to be comparable to that of the ACTICOAT dressing.

## Claims

1. A wound dressing material comprising a solid bioabsorbable substrate wherein the substrate is a freeze-dried or solvent-dried sponge and comprises a solid bioabsorbable material which is dyed with an antioxidant dyestuff and which is selected from the group consisting of oxidized regenerated cellulose, collagen, chitosan or mixtures thereof.

2. A wound dressing material according to any preceding claim, wherein the antioxidant dyestuff is selected from the group consisting of aniline dyes, acridine dyes, thionine dyes, bis-naphthalene dyes, thiazine dyes, azo dyes, anthraquinones, and mixtures thereof.

3. A wound dressing material according to any preceding claim, wherein the antioxidant dyestuff is selected from the group consisting of gentian violet, aniline blue, methylene blue, crystal violet, acriflavine, 9-aminoacridine, acridine yellow, acridine orange, proflavin, quinacrine, brilliant green, trypan blue, trypan red, malachite green, azacrine, methyl violet, methyl orange, methyl yellow, ethyl violet, acid orange, acid yellow, acid blue, acid red, thioflavin, alphazurine, indigo blue, methylene green, and mixtures thereof.

4. A wound dressing material according to any preceding claim, wherein the antioxidant dyestuff is present in an amount of from 0.2 to 2wt.% based on the dry weight of the material.

5. A wound dressing material according to any preceding claim, wherein the material further comprises a silver salt, whereby the dyestuff photostabilizes the silver salt.

6. A wound dressing material according to claim 5, wherein the polymeric substrate comprises an anionic polymer, and said silver salt comprises a salt of Ag⁺ with the anionic polymer.

7. A wound dressing material according to claim 5 or 6, wherein the composition comprises from 0.01wt.% to 5wt.% of silver, based on the dry weight of the composition.

8. A wound dressing material according to any preceding claim, wherein the material is in sheet form.

9. A wound dressing material according to any preceding claim, wherein the material is sterile and packaged in a microorganism-impermeable container.

10. A wound dressing material according to any preceding claim, wherein the material has a free radical activity in the diphenylpicrylhydrazyl (DPPH) test for antioxidant activity as herein defined of at least 15%.

11. Use of a material according to any one of claims 1 to 10 for the preparation of a medicament for the treatment of a wound.

12. Use according to claim 11, wherein the wound is a chronic wound, preferably selected from the group consisting of ulcers of venous ulcers, decubitus ulcers, or diabetic ulcers.

13. A method of manufacture of an antioxidant wound dressing material, comprising the step of dyeing a bioabsorbable substrate material with an antioxidant dye, wherein the substrate is a freeze-dried or solvent-dried sponge and comprises a solid bioabsorbable material selected from the group consisting of oxidized regenerated cellulose, collagen, chitosan or mixtures thereof.

14. A method according to claim 13 for the manufacture of a wound dressing material according to any of claims 1 to 10.

15. A wound dressing comprising an antioxidant wound dressing material according to any of claims 1 to 10.

16. A wound dressing according to claim 15, wherein the material is sterile and packaged in a microorganism-impermeable container.

## Patentansprüche

1. Wundverbandmaterial, umfassend ein festes bioresorbierbares Substrat, wobei das Substrat ein gefriergetrockneter oder lösungsmittelgetrockneter Schwamm ist und ein festes bioresorbierbares Material umfasst, das mit einem antioxidierend wirkenden Farbstoff gefärbt ist und aus der Gruppe bestehend aus oxidierter Regeneratcellulose, Kollagen, Chitosan oder Mischungen davon ausgewählt ist.

2. Wundverbandmaterial nach einem der vorhergehenden Ansprüche, wobei der antioxidierend wirkende Farbstoff aus der Gruppe bestehend aus Anilinfarbstoffen, Acridinfarbstoffen, Thioninfarbstoffen, Bisnaphthalinfarbstoffen, Thiazinfarbstoffen, Azofarbstoffen, Anthrachinonen und Mischungen davon ausgewählt ist.

3. Wundverbandmaterial nach einem der vorhergehenden Ansprüche, wobei der antioxidierend wirkende Farbstoff aus der Gruppe bestehend aus Gentianviolett, Anilinblau, Methylenblau, Kristallviolett, Acriflavon, 9-Aminoacridin, Acridingelb, Acridinorgane, Proflavin, Chinacrin, Brillantgrün, Trypanblau, Trypanrot, Malachitgrün, Azacrin, Methylviolett, Methylorange, Methylgelb, Ethylviolett, Säureorange, Säuregelb, Säuerblau, Säurerot, Thioflavon, Alphazurin, Indigoblau, Methylengrün und Mischungen davon ausgewählt ist.

4. Wundverbandmaterial nach einem der vorhergehenden Ansprüche, wobei der antioxidierend wirkende Farbstoff in einer Menge von 0,2 bis 2 Gew.-%, bezogen auf das Trockengewicht des Materials, vorliegt.

5. Wundverbandmaterial nach einem der vorhergehenden Ansprüche, wobei das Material ferner ein Silbersalz umfasst, wobei der Farbstoff das Silbersalz photostabilisiert.

6. Wundverbandmaterial nach Anspruch 5, wobei das polymere Substrat ein anionisches Polymer umfasst und das Silbersalz ein Salz von Ag⁺ mit dem anionischen Polymer umfasst.

7. Wundverbandmaterial nach Anspruch 5 oder 6, wobei die Zusammensetzung 0,01 Gew.-% bis 5 Gew.-% Silber, bezogen auf das Trockengewicht der Zusammensetzung, umfasst.

8. Wundverbandmaterial nach einem der vorhergehenden Ansprüche, wobei das Material in flächiger Form vorliegt.

9. Wundverbandmaterial nach einem der vorhergehenden Ansprüche, wobei das Material steril und in einem für Mikroorganismen undurchlässigen Behälter verpackt ist.

10. Wundverbandmaterial nach einem der vorhergehenden Ansprüche, wobei das Material eine Radikalaktivität im Diphenylpicrylhydrazyl-Test (DPPH-Test) auf Antioxidanswirkung gemäß der hier angegebenen Definition von mindestens 15% aufweist.

11. Verwendung eines Materials nach einem der Ansprüche 1 bis 10 zur Herstellung eines Arzneimittels zur Behandlung einer Wunde.

12. Verwendung nach Anspruch 11, wobei es sich bei der Wunde um eine chronische Wunde, die vorzugsweise aus der Gruppe bestehend aus Venengeschwüren, Dekubitalgeschwüren oder diabetischen Geschwüren ausgewählt ist, handelt.

13. Verfahren zur Herstellung eines antioxidierend wirkenden Wundverbandmaterials, bei dem man ein bioresorbierbares Substratmaterial mit einem antioxidierend wirkenden Farbstoff färbt, wobei das Substrat ein gefriergetrockneter oder lösungsmittelgetrockneter Schwamm ist und ein festes bioresorbierbares Material umfasst, das aus der Gruppe bestehend aus oxidierter Regeneratcellulose, Kollagen, Chitosan oder Mischungen davon ausgewählt ist.

14. Verfahren nach Anspruch 13 zur Herstellung eines Wundverbandmaterials nach einem der Ansprüche 1 bis 10.

15. Wundverband, umfassend ein antioxidierend wirkendes Wundverbandmaterial nach einem der Ansprüche 1 bis 10.

16. Wundverband nach Anspruch 15, wobei das Material steril und in einem für Mikroorganismen undurchlässigen Behälter verpackt ist.

## Revendications

1. Matériau de pansement comprenant un substrat solide bioabsorbable, le substrat étant une éponge lyophilisée ou séchée par évaporation des solvants et comprenant un matériau solide bioabsorbable qui est teinté avec un colorant antioxydant et qui est choisi dans le groupe constitué par la cellulose oxydée régénérée, le collagène, le chitosane et les mélanges de ceux-ci.

2. Matériau de pansement selon la revendication précédente, dans lequel le colorant antioxydant est choisi dans le groupe constitué par les colorants d'aniline, les colorants d'acridine, les colorants de thionine, les colorants de bis-naphtalène, les colorants de thiazine, les colorants azoïques, les anthraquinones, et les mélanges de ceux-ci.

3. Matériau de pansement selon l'une quelconque des revendications précédentes, dans lequel le colorant antioxydant est choisi dans le groupe constitué par le violet de gentiane, le bleu d'aniline, le bleu de méthylène, le cristal violet, l'acriflavine, la 9-aminoacridine, le jaune d'acridine, l'orange d'acridine, la proflavine, la quinacrine, le vert brillant, le bleu trypan, le rouge trypan, le vert de malachite, l'azacrine, le violet de méthyle, l'orange de méthyle, le jaune de méthyle, le violet d'éthyle, l'orange acide, le jaune acide, le bleu acide, le rouge acide, la thioflavine, l'alphazurine, le bleu d'indigo, le vert de méthylène, et les mélanges de ceux-ci.

4. Matériau de pansement selon l'une quelconque des revendications précédentes, dans lequel le colorant antioxydant est présent dans une quantité de 0,2 à 2 % en poids, rapporté au poids sec du matériau.

5. Matériau de pansement selon l'une quelconque des revendications précédentes, le matériau comprenant en outre un sel d'argent, le colorant photostabilisant le sel d'argent.

6. Matériau de pansement selon la revendication 5, dans lequel le substrat polymère comprend un polymère anionique, et ledit sel d'argent comprend un sel d'Ag⁺ avec le polymère anionique.

7. Matériau de pansement selon la revendication 5 ou 6, dans lequel la composition comprend de 0,01 % en poids à 5 % en poids d'argent, rapporté au poids sec de la composition.

8. Matériau de pansement selon l'une quelconque des revendications précédentes, le matériau étant sous forme de feuille.

9. Matériau de pansement selon l'une quelconque des revendications précédentes, le matériau étant stérile et conditionné dans un récipient imperméable aux microorganismes.

10. Matériau de pansement selon l'une quelconque des revendications précédentes, le matériau ayant une activité antiradicalaire dans le test au diphényl-picrylhydrazyle (DPPH) pour l'activité antioxydante tel que défini dans la description d'au moins 15 %.

11. Utilisation d'un matériau selon l'une quelconque des revendications 1 à 10 pour la préparation d'un médicament destiné au traitement d'une plaie.

12. Utilisation selon la revendication 11, dans laquelle la plaie est une plaie chronique, de préférence choisie dans le groupe constitué par les ulcères veineux, les escarres de décubitus et les ulcères diabétiques.

13. Procédé de fabrication d'un matériau de pansement antioxydant, comprenant l'étape de teinture d'un matériau de substrat bioabsorbable avec un colorant antioxydant, dans lequel le substrat est une éponge lyophilisée ou séchée par évaporation des solvants et comprend un matériau solide bioabsorbable choisi dans le groupe constitué par la cellulose oxydée régénérée, le collagène, le chitosane et les mélanges de ceux-ci.

14. Procédé selon la revendication 13 pour la fabrication d'un matériau de pansement selon l'une quelconque des revendications 1 à 10.

15. Pansement comprenant un matériau de pansement antioxydant selon l'une quelconque des revendications 1 à 10.

16. Pansement selon la revendication 15, dans lequel le matériau est stérile et conditionné dans un récipient imperméable aux microorganismes.
